# EUROPEAN PATENT APPLICATION

(11) **EP 4 245 262 A1**
(43) Date of publication of application: **20.09.2023**
(21) Application number: 22784790.2
(22) Date of filing: 11.03.2022
(51) Int. Cl.: A61F 2/30, A61L 27/56

(54) **AUGMENT IMPLANT TO WHICH DIFFERENT TYPES OF POROUS STRUCTURES ARE APPLIED**

(30) Priority: 07.04.2021 KR 20210045237
(71) Applicant: Corentec Co., Ltd., Seoul 06649 (KR)
(72) Inventor: KIM, Jung-Sung, Seoul 05555 (KR); KANG, Yeo-Kyung, Seongnam-si Gyeonggi-do 13162 (KR); KIM, Seong-Jin, Seoul 06640 (KR)
(74) Representative: Witte, Weller & Partner Patentanwälte mbB
(86) International application number: PCT/KR2022/003474
(87) International publication number: WO 2022/215880

(57) **Abstract**

Proposed is an augment implant to which different types of porous structures are applied and, more specifically, an augment implant to which different types of porous structures are applied in which a porous structure of an irregular shape is formed on the outer side of the augment implant and a porous structure of a regular shape is formed on the inner side of the augment implant, thereby ensuring an initial fixing power by means of a rough surface, inducing bone ingrowth to provide a long-term biological fixing power, making the augment implant lightweight, enabling a structure thereof to be shaped with a remarkably low amount of data, reinforcing the strength of a body part having a porous structure through a frame part having a nonporous structure, and facilitating the discharge of remaining inner powder through a powder hole.

## Description

### Technical Field

The present disclosure relates to an augment implant to which different types of porous structures are applied and, more specifically, to an augment implant to which different types of porous structures are applied in which a porous structure of an irregular shape is formed on the outer side of the augment implant and a porous structure of a regular shape is formed on the inner side of the augment implant, thereby ensuring an initial fixing power by means of a rough surface, inducing bone ingrowth to provide a long-term biological fixing power, making the augment implant lightweight, enabling a structure thereof to be shaped with a remarkably low amount of data so that the manufacturing costs of the augment implant are lowered due to reduction in time, labor and costs required in manufacturing, reinforcing the strength of a body part having a porous structure through a frame part having a nonporous structure, and facilitating the discharge of remaining inner powder through a powder hole.

### Background Art

Artificial joint replacement is performed in a process of manufacturing an implant having a shape complementary to the anatomical shape of a damaged joint, cutting the bone of the damaged joint within a predetermined range, and implanting the implant in the cut area. In this case, when bone cutting is required entirely or partially, such as when the damaged joint area is extensive, has severe local damage, or requires revision surgery, an augment implant may be used to compensate for a bone area lost by cutting.

The augment implant is a component which supplements a lost bone, and is formed to have a shape complementary to the shape of the lost bone area, so that the augment implant is seated in empty space defined by the cut bone. In general, such an augment implant may be made of biomaterials (biometals, bioceramics, biopolymers) in a solid form.

However, when the augment implant is configured in a solid form without a pore, a bone cannot grow in an area equal to space occupied by the augment implant. For this reason, a method of forming the augment implant with a porous material having multiple pores has been sought.

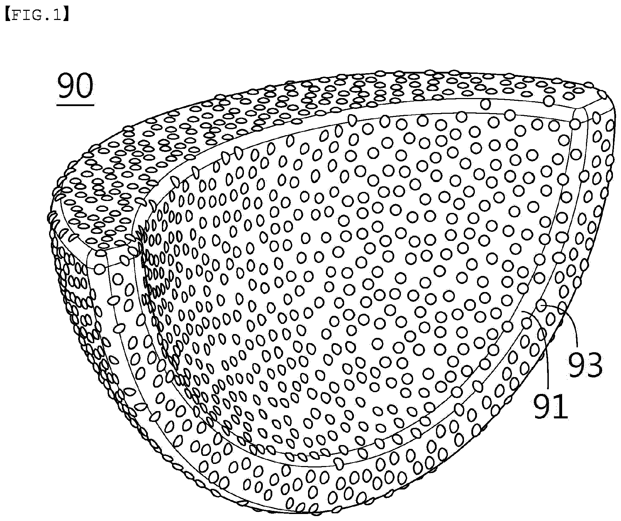

FIG. 1 is a view of a conventional porous augment implant 90, which is disclosed in Korean Patent Application Publication No. 10-2014-0031828 (published on March 13, 2014) .

The conventional porous augment implant 90 includes a first porous surface 91 in contact with a patient's orthopedic joint, and the first porous surface 91 has a plurality of pores sized to enable bone ingrowth in the augment implant. The augment implant further includes a plurality of protrusions 93 which is connected to the first porous surface 91 and protrudes as tall as 200µm to 400µm on the first porous surface 91. The plurality of protrusions 93 is configured to create friction with a bone surface without extending into a bone surface and without interfering with bone ingrowth into the plurality of pores. According to the conventional technology 90, a patient's autogenous bone growth can be promoted due to the first porous surface 91 having multiple pores, so the augment implant and the patient's bone can be strongly combined with each other.

However, as in the conventional technology 90, when the augment implant is formed with only a porous material having multiple pores, a patient's autogenous bone growth can be promoted, but due to many pores formed in the augment implant, the mechanical strength of the augment implant is decreased. That is, the porous augment implant having a relatively low strength may be easily damaged, such as being broken, cracked, or crushed even by a small external force. In addition, in a process in which a porous part is damaged, porous particles, which are fragments of the porous part, may be generated. When these porous particles are introduced into the pores of the porous part, the porous particles may not be completely removed even after washing. When the augment implant from which fine particles are not removed is implanted into a patient's body, the fine particles may come off from the implanted porous augment implant, and infiltrate the patient's blood vessels, and the internal tissues of the body, etc., which may cause problems such as various inflammatory reactions and tissue necrosis. Furthermore, the augment implant is often combined with an implant to be implanted together, and when a screw used in the process of combining the augment implant with the implant presses the porous augment implant, the porous augment implant having low strength is easily damaged by the screw, and due to this, a strong fixing force between the augment implant and the implant cannot be sufficiently secured.

In some cases, bone cement was used to solve this problem, but in this case, the problem of leakage of the bone cement leakage occurred. It has been reported that due to this, there occurred osteolysis, a phenomenon of a bone melting at a portion at which the implant and the patient's bone are in contact with each other. Furthermore, when bone cement is used, a large part of pores of a porous part was clogged. Accordingly, the porous part made to promote a patient's natural bone growth was clogged by the bone cement and failed to promote bone ingrowth.

In addition, the conventional porous augment implant was manufactured by an additive manufacturing method called 3D printing. When there is empty space in an output printed by a 3D printer, a support which is a means which supports the output is required. In the absence of the support, a 3D printer laminates materials in the air. In order to print an output having a desired shape, an appropriate arrangement of the support is required to be designed.

However, as illustrated in FIG. 1, when the augment implant is formed as a structure having pores formed in the entirety of the augment implant, the support is unavoidably attached to a porous part having relatively low strength, and thus when removing the support from the porous augment implant after printing the support, even the porous part which is required to be prevented from being removed, together with the support, is removed, so the defective rate of finished products was high. Accordingly, a 3D printing manufacturing process is required to be performed again, thereby resulting in unnecessary waste of time, cost, and effort.

In addition, in a case in which a porous structure with an irregular and complex shape is designed when using 3D printing to form the porous augment implant to have the entire shape of a porous structure, the size of the data capacity of design files for 3D printing is greatly increased, resulting in excessive manufacturing costs and time.

Accordingly, in the related industry, there is a demand for the development of a new type of augment implant which has a porous structure for maximizing a patient's bone growth to replace the area of a damaged bone together with an artificial acetabular cup, can be lightweight, is not easily damaged due to the protection of a porous part and the reinforcement of strength, allows a support necessary during the manufacturing of the augment implant by 3D printing to be easily removed, and enables the data capacity of design files for 3D printing to be reduced.

(Patent Document 1) Korean Patent Application Publication N. 10-2014-0031828 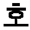 (2014.03.13.)

### Disclosure

### Technical Problem

The present disclosure has been made to solve above problems, and
an objective of the present disclosure is to propose an augment implant which provides stability and relieves a patient's pain by compensating for a missing bone in response to a damaged bone requiring hip replacement surgery.

Another objective of the present disclosure is to propose an augment implant in which a body part of a porous structure is formed so as to induce bone ingrowth through pores of the body part.

Still another objective of the present disclosure is that the body part of a porous structure includes a plurality of porous parts having porosities different from each other so that autogenous bone ingrowth is promoted and the weight of an implant is reduced.

Still another objective of the present disclosure is that a first porous part constituting the outer side of the body part and a second porous part constituting the inner side of the body part are formed, and the porosity of the first porous part is smaller than the porosity of the second porous part, so that along with the generation of an initial fixing power in a bone contact area, a long-term biological fixing power is generated due to bone ingrowth through the first porous part, and the second porous part located inside the first porous part induces bone ingrowth and lightens an implant.

Still another objective of the present disclosure is that the size of a pore of the first porous part is smaller than the size of a pore of the second porous part, and thus the outer side of the body part is smaller in porosity and the size of a pore than the inner side of the body part so as to relatively increase the strength of the outer side of the body part, thereby decreasing damage and occurrence of fine fragments.

Still another objective of the present disclosure is that the porous structure of the first porous part has an irregular shape so that after the insertion of an implant, an initial fixing power thereof is secured by friction due to high surface roughness.

Still another objective of the present disclosure is that an initial fixing power of the augment implant is generated due to high friction caused by the irregular first porous part, and a long-term biological fixing power is generated through bone ingrowth in a bone contact area.

Still another objective of the present disclosure is that the porous structure of the second porous part has a regular shape so that time, efforts, and costs required for the manufacturing of the second porous part are reduced compared to the first porous part so as to reduce the manufacturing costs of the implant.

Still another objective of the present disclosure is that the second porous part has a regular porous structure so that a structure thereof can be shaped with significantly less data compared to the first porous part.

Still another objective of the present disclosure is that the inside of the augment implant has the shape of a regular and simple porous structure to significantly reduce time required for slicing necessary to manufacture the augment implant by 3D printing so that the manufacturing expenses and costs of the augment implant can be reduced.

Still another objective of the present disclosure is that the inside of the augment implant has the shape of a regular and simple porous structure so that the capacity of data can be greatly reduced during designing.

Still another objective of the present disclosure is that a wall frame part of a nonporous structure is formed along a boundary surface between the first porous part and the second porous part which are porous structures so that an implant is prevented from being damaged due to reinforced strength, and the first porous part of an irregular porous structure and the second porous part of a regular porous structure are prevented from being separated from each other.

Still another objective of the present disclosure is that a through hole is formed through a second surface of the body part from a first surface of the body part, and a hole frame part having a nonporous structure is formed along the inner surface of the through hole so that the body part having low strength around the through hole is prevented from being damaged by a fixing means inserted into the through hole.

Still another objective of the present disclosure is that a powder hole is formed through a second surface of the hole frame part from a first surface of the hole frame part so that remaining powder present inside the augment implant manufactured by 3D printing can be easily discharged to the outside to be removed.

Still another objective of the present disclosure is that a plurality of powder holes is formed in the hole frame part so that remaining powder can be more easily removed when manufacturing the augment implant by 3D printing of a PBF method.

Still another objective of the present disclosure is that although remaining powder of the first porous part having an irregular structure located on the outer side of the body part can be removed through the pores of the first porous part, the removal of remaining powder of the second porous part which is a regularly-shaped structure is difficult since the second porous part is located inside the first porous part, and thus when forming the powder hole in the hole frame part, the powder hole is formed at a position at which the powder hole communicates with the second porous part so that the remaining powder of the second porous part can be removed through the powder hole formed in the hole frame part.

Still another objective of the present disclosure is that the through hole includes a first tapered hole which has a truncated cone-shaped space by having a diameter decreasing gradually toward a second side from a first side, and a second tapered hole which faces the first tapered hole and has a truncated cone-shaped space by having a diameter decreasing gradually toward the first side from the second side so that when inserting the fixing means such as a screw for fixing the augment implant into the through hole, the fixing means is inserted at various angles along a tapered inclined surface of the through hole, thereby enabling the augment implant to be fixed in various directions suitable for a patient's condition.

Still another objective of the present disclosure is that a linear edge frame part is formed on an edge which is a boundary between a surface and a surface in the body part so as to protect the body part and increase the strength of the augment implant, and when the augment implant is manufactured by 3D printing, a support is connected to the edge frame part so that in the process of removing the support, the support is easily removed without damage to a porous structure.

Still another objective of the present disclosure is that the augment implant, in which the frame part of a non-porous structure is combined with the body part of a porous structure, is integrally manufactured by 3D printing.

Still another objective of the present disclosure is that a porous structure having micro-sized pores is accurately and easily manufactured by using 3D printing.

### Technical Solution

In order to accomplish the above objectives, an augment implant of the present disclosure having the following components is realized according to embodiments.

According to one embodiment of the present disclosure, the augment implant of the present disclosure includes: a body part of a porous structure which has pores which induce bone ingrowth, wherein the body part includes a plurality of porous parts having porosities different from each other.

According to another embodiment of the present disclosure, the body part may include a first porous part constituting an outer side of the body part, and a second porous part constituting an inner side of the body part.

According to still another embodiment of the present disclosure, a porosity of the first porous part may be smaller than a porosity of the second porous part.

According to still another embodiment of the present disclosure, a size of a pore of the first porous part may be smaller than a size of a pore of the second porous part.

According to still another embodiment of the present disclosure, a porous structure of the first porous part may be formed to have an irregular shape.

According to still another embodiment of the present disclosure, a porous structure of the second porous part may be formed to have a regular shape.

According to still another embodiment of the present disclosure, the augment implant may further include a wall frame part of a nonporous structure formed along a boundary surface between the first porous part and the second porous part.

According to still another embodiment of the present disclosure, the body part may include a through hole formed through a second surface of the body part from a first surface thereof, and the augment implant may further include a hole frame part of a nonporous structure formed along an inner surface of the through hole.

According to still another embodiment of the present disclosure, the hole frame part may include a powder hole formed through a second surface of the hole frame part from a first surface thereof.

According to still another embodiment of the present disclosure, the powder hole may be formed at a position at which the powder hole communicates with the second porous part of the body part.

According to still another embodiment of the present disclosure, the through hole may include a first tapered hole which has a truncated cone-shaped space by having a diameter decreasing gradually toward a second side from a first side, and a second tapered hole which faces the first tapered hole and has a truncated cone-shaped space by having a diameter decreasing gradually toward the first side from the second side so that multidirectional fixing of a fixing means is possible.

According to still another embodiment of the present disclosure, the body part may include an edge which is a boundary between a surface and a surface, and the augment implant may further include an edge frame part of a nonporous structure formed along the edge.

According to still another embodiment of the present disclosure, the augment implant may be integrally manufactured by 3D printing.

### Advantageous Effects

The present disclosure can have the following effects by the above embodiments, components to described below, combination thereof, and use relationship thereof.

According to the present disclosure, a missing bone is compensated in response to a damaged bone requiring hip replacement surgery, thereby providing the augment implant providing stability and relieving a patient's pain.

According to the present disclosure, the body part of a porous structure is formed, thereby providing the augment implant inducing bone ingrowth through pores of the body part.

According to the present disclosure, the body part of a porous structure includes the plurality of porous parts having porosities different from each other, thereby promoting autogenous bone ingrowth and enabling weight reduction of an implant.

According to the present disclosure, a first porous part constituting the outer side of the body part and a second porous part constituting the inner side of the body part are formed, and the porosity of the first porous part is smaller than the porosity of the second porous part, thereby a long-term biological fixing power is generated due to bone ingrowth through the first porous part along with the generation of an initial fixing power in a bone contact area, and enabling bone ingrowth to be induced and an implant to be light by the second porous part located inside the first porous part.

According to the present disclosure, the size of a pore of the first porous part is smaller than the size of a pore of the second porous part, and thus the outer side of the body part is smaller in porosity and the size of a pore than the inner side of the body part so as to relatively increase the strength of the outer side of the body part, thereby decreasing damage and occurrence of fine fragments.

According to the present disclosure, the porous structure of the first porous part has an irregular shape, thereby securing an initial fixing power by friction due to high surface roughness after the insertion of an implant.

According to the present disclosure, an initial fixing power of the augment implant is generated due to high friction caused by the irregular first porous part, thereby generating a long-term biological fixing power through bone ingrowth in a bone contact area.

According to the present disclosure, the porous structure of the second porous part has a regular shape so that time, efforts, and costs required for the manufacturing of the second porous part are reduced compared to the first porous part, thereby reducing the manufacturing costs of the implant.

According to the present disclosure, the second porous part has a regular porous structure, thereby shaping a structure thereof with significantly less data compared to the first porous part.

According to the present disclosure, the inside of the augment implant has the shape of a regular and simple porous structure to significantly reduce time required for slicing necessary to manufacture the augment implant by 3D printing, thereby reducing the manufacturing expenses and costs of the augment implant.

According to the present disclosure, the inside of the augment implant has the shape of a regular and simple porous structure, thereby greatly reducing the capacity of data during designing.

According to the present disclosure, the wall frame part of a nonporous structure is formed along a boundary surface between the first porous part and the second porous part which are porous structures, thereby preventing the implant from being damaged due to reinforced strength, and preventing the first porous part of an irregular porous structure and the second porous part of a regular porous structure from being separated from each other.

According to the present disclosure, the through hole is formed through the second surface of the body part from the first surface of the body part, and the hole frame part having a nonporous structure is formed along the inner surface of the through hole, thereby preventing the body part having low strength around the through hole from being damaged by a fixing means inserted into the through hole.

According to the present disclosure, the powder hole is formed through the second surface of the hole frame part from the first surface of the hole frame part, thereby easily discharging remaining powder present inside the augment implant manufactured by 3D printing to the outside to be removed.

According to the present disclosure, a plurality of powder holes is formed in the hole frame part, thereby more easily removing remaining powder when manufacturing the augment implant by 3D printing of a PBF method.

According to the present disclosure, although remaining powder of the first porous part of an irregular structure located on the outer side of the body part can be removed through the pores of the first porous part, the removal of remaining powder of the second porous part which is a regularly-shaped structure is difficult since the second porous part is located inside the first porous part, and thus when forming the powder hole in the hole frame part, the powder hole is formed at a position at which the powder hole communicates with the second porous part, thereby removing the remaining powder of the second porous part through the powder hole formed in the hole frame part.

According to the present disclosure, the through hole includes the first tapered hole which has a truncated cone-shaped space by having a diameter decreasing gradually toward a second side from a first side, and the second tapered hole which faces the first tapered hole and has a truncated cone-shaped space by having a diameter decreasing gradually toward the first side from the second side so that when inserting the fixing means such as a screw for fixing the augment implant into the through hole, the fixing means is inserted at various angles along a tapered inclined surface of the through hole, thereby enabling the augment implant to be fixed in various directions suitable for a patient's condition.

According to the present disclosure, the linear edge frame part is formed on an edge which is a boundary between a surface and a surface in the body part so as to protect the body part and increase the strength of the augment implant, and when the augment implant is manufactured by 3D printing, the support is connected to the edge frame part, thereby facilitating the removal of the support without damage to a porous structure in the process of removing the support.

According to the present disclosure, the augment implant in which the frame part of a non-porous structure is combined with the body part of a porous structure can be integrally manufactured by 3D printing.

According to the present disclosure, a porous structure having micro-sized pores can be accurately and easily manufactured by using 3D printing.

### Description of Drawings

FIG. 1 is a view illustrating a conventional porous augment implant.
FIG. 2 is a view illustrating an augment implant to which different types of porous structures are applied according to an embodiment of the present disclosure viewed from an aspect.
FIG. 3 is a view illustrating the augment implant to which different types of porous structures are applied according to the embodiment of the present disclosure viewed from another aspect.
FIG. 4 is a view illustrating a body part of the present disclosure.
FIG. 5 is a sectional view taken along line B-B' of FIG. 4.
FIG. 6 is a sectional view taken along line A-A' of FIG. 2.
FIG. 7 is a view illustrating a state in which a support is coupled to a frame part.
FIG. 8 is a view illustrating a state in which the augment implant of the present disclosure is used.

### Best Mode

Hereinbelow, exemplary embodiments of an augment implant to which different types of porous structures are applied according to the present disclosure will be described in detail with reference to the accompanying drawings. In the following description of the present disclosure, when it is determined that a detailed description of a known function or configuration may unnecessarily obscure the gist of the present disclosure, detailed description thereof will be omitted. Unless there is a special definition, all terms in this specification are the same as the general meaning of terms understood by those skilled in the art to which the present disclosure belongs, and when conflicting with the meaning of terms used in this specification, the terms used generally in the art follow the definition of the terms used herein.

In the augment implant 1 of the present disclosure to which different types of porous structures are applied, a porous structure of an irregular shape is formed on the outer side of the augment implant and a porous structure of a regular shape is formed on the inner side of the augment implant, thereby ensuring an initial fixing power by means of a rough surface, inducing bone ingrowth to provide a long-term biological fixing power, making the augment implant lightweight, enabling a structure thereof to be shaped with a remarkably low amount of data so that the manufacturing costs of the augment implant are lowered due to reduction in time, labor and costs required in manufacturing, reinforcing the strength of a body part having a porous structure through a frame part having a nonporous structure, and facilitating the discharge of remaining inner powder through a powder hole.

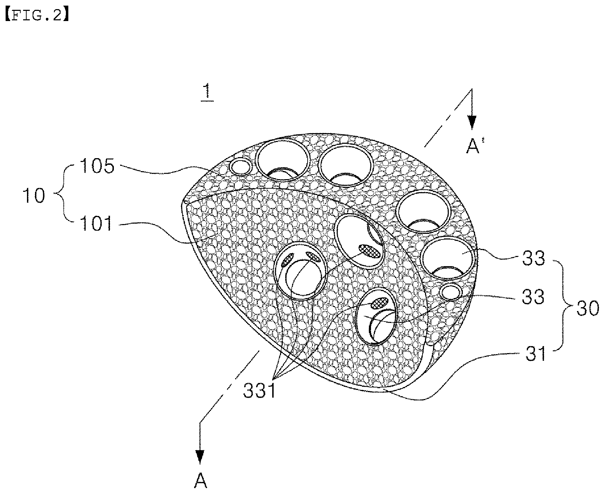

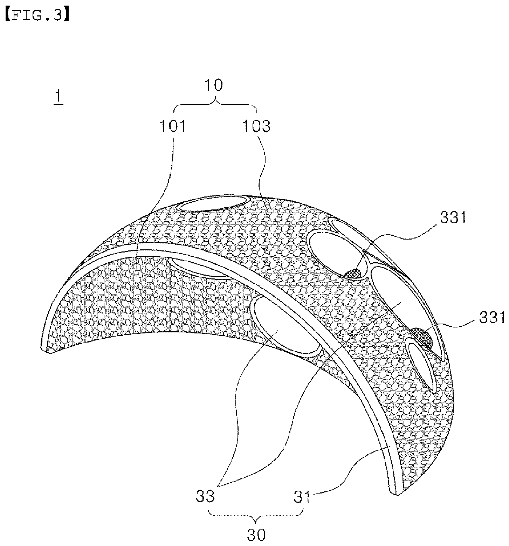

FIG. 2 is a view illustrating the augment implant 1 to which different types of porous structures are applied according to an embodiment of the present disclosure viewed from an aspect, and FIG. 3 is a view illustrating the augment implant 1 to which different types of porous structures are applied according to the embodiment of the present disclosure viewed from another aspect. Referring to FIGS. 2 and 3, the augment implant 1 to which different types of porous structures are applied includes the body part 10 and the frame part 30.

The body part 10 is a component having a shape complementary to the shape of an area of a damaged bone and as illustrated in the drawings, is configured as a porous structure having many pores which induce bone ingrowth. Preferably, the body part 10 may include a plurality of porous parts having porosities different from each other. Hereinafter, the body part 10 has been described to include two types of a first porous part 15 and a second porous part 17, but may include more porous parts having a different porosity for each part as needed. The body part 10 of a porous structure which has multiple pores is formed to increase surface roughness so that the body part 10 can be stably seated on a bone, and bone ingrowth can be promoted through the multiple pores. Preferably, a porous part having a relatively high porosity may be located on the inner side of the body part 10, and a porous part having a relatively low porosity may be located on the outer side of the body part 10.

The body part 10 is not limited to any specific shape, and may be configured to have a shape suitable for filling a portion of a bone in which bone damage or defect has occurred. To this end, the body part 10 may be customized for a patient through the process of measuring and modeling a bone part of a patient requiring the augment implant 1 by reconstructing medical images such as MRI or CT, and through a 3D printing process thereof.

The body part 10 is preferably formed to have a shape complementary to the shape of a patient's missing bone. The body part 10 has a surface, and referring to FIGS. 2 and 3, the surface may be divided into an implant contact surface 101 which is a surface in contact with the implant prosthesis, a bone contact surface 103 which is a surface in contact with a bone, and a connecting surface 105 which connects the implant contact surface 101 with the bone contact surface 103. The augment implant 1 of the present disclosure may be preferably used for hip joint revision surgery, and is combined with the side of an acetabular cup, which is an implant prosthesis inserted into a patient's acetabular, to rebuild a damaged bone. To this end, the implant contact surface 101 of the body part 10 may be configured to have a shape complementary to an outer circumferential surface of the implant prosthesis.

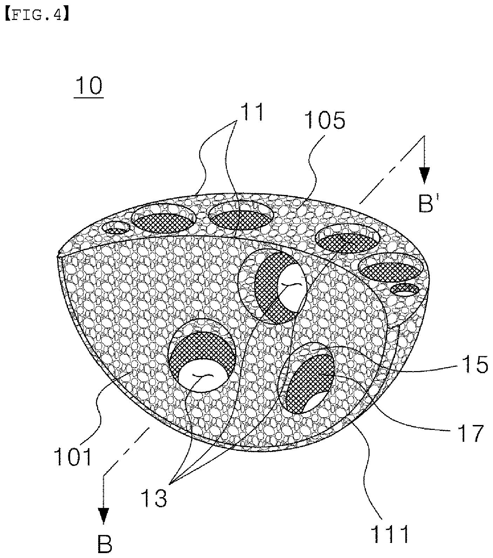

FIG. 4 is a view illustrating the body part 10 of the present disclosure, and the body part 10 includes an edge 11, a through hole 13, the first porous part 15, and the second porous part 17.

The edge 11 means a boundary at which different surfaces of the body part 10 meet each other, and may be considered to mean a cornered edge of an object. That is, the edge 11 may be formed in a boundary between the implant contact surface 101 and the bone contact surface 103, a boundary between the bone contact surface 103 and the connecting surface 105, and a boundary between the connecting surface 105 and the implant contact surface 101.

The edge 11 includes a contact edge 111, and the contact edge 111 means a boundary between the implant contact surface 101 of the body part 10 which is a surface in contact with an implant and the bone contact surface 103 of the body part 10 which is a surface in contact with a bone. Preferably, the contact edge 111 may be formed as a sharp edge which minimizes empty space between the implant A and the implant contact surface 101. A sharp edge has concept opposite to a round edge, and in a case in which the contact edge 111 is formed to have a round edge having a round end, when an implant and the augment implant 1 are closely coupled to each other, there may be a lot of empty space therebetween. In order to solve this problem, in the augment implant of the present disclosure, the contact edge 111 is formed as a sharp edge having an end which is not round, and thus when the augment implant 1 is in contact with an implant A, the creation of empty space between the implant and the augment implant 1 may be more minimized.

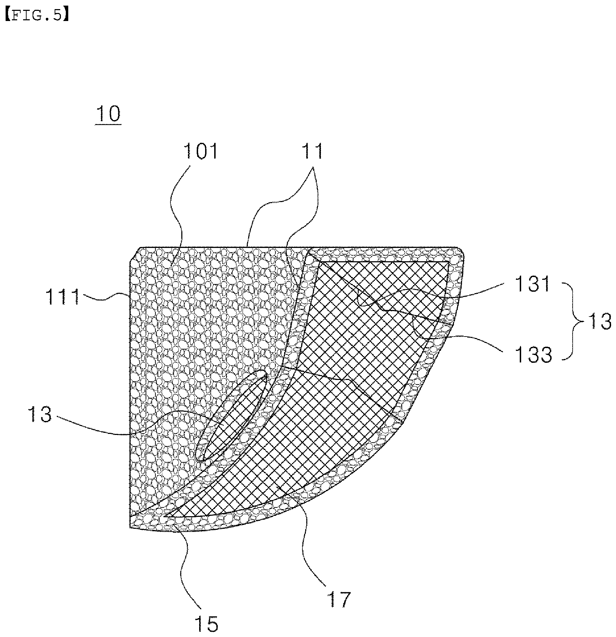

The through hole 13 refers to a hole formed through a second surface of the body part 10 from a first surface thereof, and provides space into which a fixing means such as a screw can be inserted. As illustrated in FIGS. 4 and 5, the through hole 13 may be formed through the bone contact surface 103 from the implant contact surface 101 of the body part 10, and may be formed through the bone contact surface 103 from the connecting surface 105 of the body part 10. The through hole 13 may be preferably formed to have a circular cross-sectional shape so that the fixing means is easily inserted into the through hole 13. In addition, to secure the fixing power of the augment implant 1, the through hole 13 may include a plurality of through holes formed in the body part 10.

FIG. 5 is a sectional view taken along line B-B' of FIG. 4. Referring to FIG. 5, the through hole 13 includes a first tapered hole 131, and a second tapered hole 133.

The first tapered hole 131 refers to a component which has a truncated cone-shaped space by having a diameter decreasing gradually toward the second side from the first side. An inclined surface is formed by the first tapered hole 131, and the fixing means guided along the inclined surface, such as a screw or bone cement, may be inserted at various angles and be fixed.

The second tapered hole 133 refers to a component which faces the first tapered hole 131 and has a truncated cone-shaped space by having by having a diameter decreasing gradually toward the first side from the second side. An inclined surface is formed even at a side opposite to the first tapered hole 131 by the second tapered hole 133, and thus in addition to the multidirectional fixing of the fixing means toward the second side from the first side, the multidirectional fixing of the fixing means toward the first side from the second side may be performed. Although not shown, a connection hole may be formed between the first tapered hole 131 and the second tapered hole 133, and the first tapered hole 131 and the second tapered hole 133 may be configured to communicate with each other through the connection hole.

The first porous part 15 is a component constituting the outer side of the body part 10, and the porous structure of the first porous part 15 may preferably have an irregular shape so that an initial fixing power is secured due to friction caused by high surface roughness after the insertion of the augment implant. The porosity of the first porous part 15 is smaller than the porosity of the second porous part 17 to be described later, and the size of a pore of the first porous part 15 is smaller than the size of a pore of the second porous part 17 to be described later, so that the weight reduction of the manufactured augment implant 1 can be promoted. Preferably, the size of a pore of the first porous part 15 may be 750 µm, and a porosity thereof may be 60% or more. Referring to FIG. 5, the first porous part 15 may have constant thickness and may be formed on the outer side of the second porous part 17 to be described later.

The second porous part 17 is a component constituting the inner side of the body part 10, and may be formed inside the first porous part 15. Preferably, the porous structure of the second porous part 17 has a regular shape, and thus time, efforts, and costs required for manufacturing the same are reduced compared to the first porous part 15, so the manufacturing costs of the augment implant 1 can be reduced. That is, the second porous part 17 has a regular porous structure, so the structure thereof can be shaped with significantly less data compared to the first porous part 15, time required for slicing necessary for manufacturing the augment implant 1 by 3D printing can be significantly reduced to reduce the manufacturing expenses and costs of the augment implant 1, and the capacity of data during designing can be greatly reduced. Preferably, the size of a pore of the second porous part 17 may be 3, 000 µm, and a porosity thereof may be 70% or more. The pore of the second porous part 17 may communicate with the powder hole 331 of a hole frame part 33 to be described later, and through this, in the augment implant 1 manufactured by 3D printing, powder remaining in the second porous part 17 may be easily discharged to the outside.

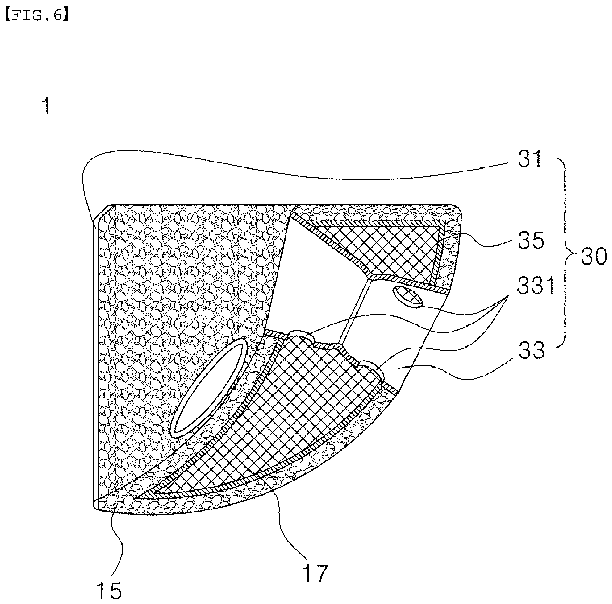

FIG. 6 is a sectional view taken along line A-A' of FIG. 2. Hereinafter, description of the augment implant will be made with reference to FIG. 6.

The frame part 30, which has a shape to prevent damage to the body part 10 and is combined with the body part 10, is formed as the non-porous structure having higher strength than the porous structure. The non-porous structure, which has a concept opposite to the porous structure, may include a structure having no pore, and may include a structure of lower porosity than the porous structure in that the frame part 30, which reinforces the strength of the body part 10 and protects the porous structure, may have strength increasing as porosity decreases. The frame part 30 of the non-porous structure is provided to protect the body part 10 of a porous structure having a relatively low strength and increase the mechanical strength of the body part 10, thereby preventing damage to the body part 10 due to external forces and minimizing decrease of porosity of the body part 10.

More preferably, the frame part 30 does not close any one surface of the body part 10 so as to enable the multidirectional insertion of the body part 10. That is, even if the frame part 30 of a non-porous structure is combined with the body part 10 of a porous structure, the frame part 30 does not close any one surface of the body part 10, so the augment implant 1 may be inserted in various directions, not just in a specific direction. When the frame part 30 closes one surface of the body part 10, bone ingrowth may not be induced in the associated one surface, and because of this, the associated surface may not be the bone contact surface which is required to have strong fixing force. However, even if the frame part 30 is formed on the body part 10, any one surface of the body part 10 is prevented from being blocked by the frame part 30. Accordingly, since penetration of a bone into the body part can be performed in all directions, the augment implant 1 and a patient's bone can be strongly combined with each other after surgery.

As described above, in order to easily manufacture the augment implant 1 including a porous structure having a complicated shape, the augment implant 1 is preferably manufactured integrally by 3D printing. However, a method of stacking materials is used in a printing process by 3D printing, and thus when there is empty space in an output printed by a 3D printer, a means for supporting the output is required. Without the support means, the 3D printer may eject a material into the air, and the printed output may sag. The augment implant 1 of the present disclosure has a shape complementary to the anatomical shape of a patient, and in order to realize an atypical augment implant 1 in a pre-planned shape by 3D printing, it is necessary to print a support S supporting the atypical augment implant 1 together with the atypical augment implant 1.

The support S is finally removed from a completed augment implant 1. When the support S is combined with the body part 10 having a porous structure, in a process of removing the support S, the body part 10 having a relatively low strength may be removed together with the support S. In this case, the augment implant 1 is required to be reprinted and the waste of costs and time occurs.

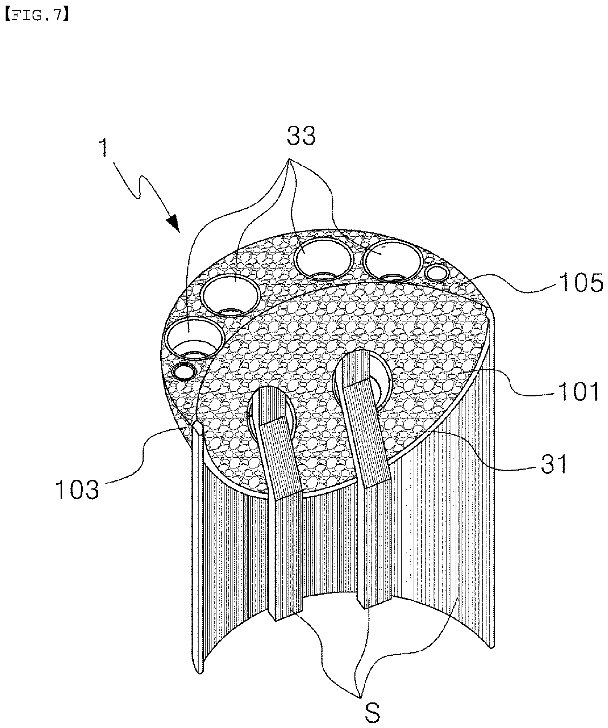

Accordingly, the augment implant 1 of the present disclosure is manufactured integrally by 3D printing, and the support S generated during 3D printing is connected to the frame part 30 as illustrated in FIG. 7. Accordingly, even if the support S is printed while being attached to the augment implant 1, the support S can be easily removed from the frame part 30 having relatively high strength and a smooth surface.

Referring to FIG. 6, the frame part 30 includes an edge frame part 31, the hole frame part 33, and a wall frame part 35.

The edge frame part 31 refers to the frame part 30 of the non-porous structure formed on the contact edge 111. The edge frame part 31 is formed along the contact edge 111 which is a boundary between the implant contact surface 101 of the body part 10 which is a surface in contact with an implant and the bone contact surface 103 of the body part 10 which is a surface in contact with a bone so that the end part of the body part 10 having low strength is prevented from being damaged, and when the augment implant 1 is manufactured by 3D printing, the support S is connected to the edge frame part 31 so that in the process of removing the support, the support is easily removed without damage to the porous structure. Above all, the linear edge frame part 31 is formed on an edge which is a boundary between a surface and a surface in the body part 10 to minimize the area of the frame part 30 and maximizing the area of the body part 10 so that bone growth can be maximized, and the body part 10 has high porosity in various directions so that bone growth can be promoted in any direction.

The hole frame part 33 is a frame part 30 formed inside the through hole 13 and is formed in a tubular shape having open first and second surfaces as illustrated in FIG. 6 so as to cover the inner surface of the through hole 13. When the fixing means is inserted into the through hole 13 to fix the augment implant 1 through the hole frame part 33, the body part 10 around the through hole 13 can be prevented from being damaged by external forces. It is preferable that the outer surface of the hole frame part 33 has a shape complementary to the shape of the inner surface of the through hole 13. Through this, the hole frame part 33 can be stably fixed in the through hole 13 to protect the through hole 13 and can be securely coupled to the fixing means. The hole frame part 33 may preferably be formed as many as the number of the through holes 13.

The hole frame part 33 includes the powder hole 331 as illustrated in FIG. 6.

The powder hole 331 is formed through a second surface of the hole frame part 33 from a first surface thereof, and may be preferably, at a position at which the powder hole 331 communicates with the second porous part 17 of the body part 10. Remaining powder present inside the augment implant 1 manufactured by 3D printing may be easily discharged through the powder hole 331 to the outside to be removed. The powder hole 331 has a plurality of powder holes formed in the hole frame part 33. Preferably, when the augment implant is manufactured by 3D printing of a powder bed fusion (PBF) method, remaining powder may be more easily removed. Remaining powder of the first porous part 15 as an irregular structure located on the outer side of the body part 10 may be removed through the pores of the first porous part 15, but the second porous part 17 which is a regularly-shaped structure is located inside the first porous part 15 and thus the removal of remaining powder in the second porous part is difficult. Accordingly, when forming the powder hole 331 in the hole frame part 33, the powder hole 331 is formed at a position at which the powder hole 331 communicates with the second porous part 17, and thus the remaining powder of the second porous part 17 is preferably removed through the powder hole 331 formed in the hole frame part 33.

The wall frame part 35 refers to the frame part 30 having a nonporous structure formed along a boundary surface between the first porous part 15 and the second porous part 17. The wall frame part 35 of a nonporous structure is formed on the boundary surface between the first porous part 15 and the second porous part 17 which are porous structures, thereby preventing damage to the augment implant 1 by reinforcing the strength of the augment implant 1, and preventing the separation of the first porous part 15 of an irregular porous structure and the second porous part 17 of a regular porous structure from each other. The wall frame part 35 may be formed of a thin film having constant thickness as illustrated in FIG. 6.

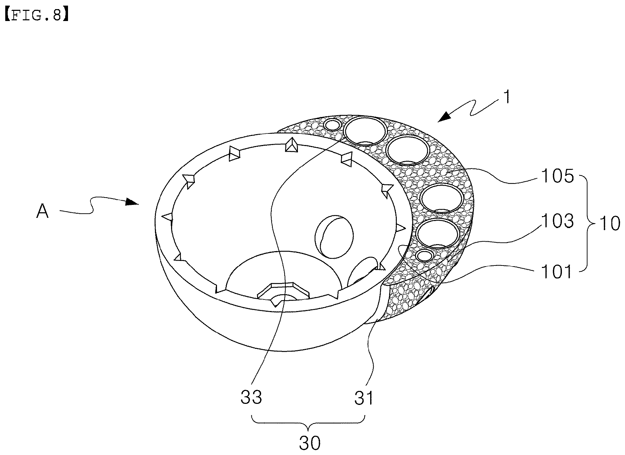

FIG. 8 is a view illustrating a state in which the augment implant of the present disclosure is used. Referring to FIG. 8, the augment implant 1 to which different types of porous structures are applied according to the present disclosure may be used for hip revision surgery, etc. and may be combined with the side of the acetabular cup A, which is an implant prosthesis inserted into the patient's acetabular, to reconstruct a damaged bone. In the augment implant 1 to which different types of porous structures are applied, the body part 10 of a porous structure which promotes bone ingrowth includes the first porous part 15 constituting the outer side of the body part 10, and the second porous part 17 constituting the inner side of the body part 10. The first porous part 15 may have high surface roughness by having an irregular structure and may provide an initial fixing power through friction. On the other hand, the second porous part 17 may have a regularly-shaped structure such as a lattice to reduce the manufacturing costs of an implant. The wall frame part 35 is formed between the first porous part 15 and the second porous part 17 to reinforce strength and prevent separation or breakage between structures. The hole frame part 33 is formed around the through hole 13 of the body part 10 to protect the through hole 13, and the powder hole 331 is formed in the hole frame part 33 so that remaining powder can be easily discharged through the powder hole 331. The augment implant 1 to which different types of porous structures are applied may be manufactured by 3D printing. During 3D printing, the support is designed to be combined with the frame part 30 as illustrated in FIG. 7, and thus in the process of removing the support, the body part 10 having relatively low strength is prevented from being broken or from being removed together with the support.

The above detailed description is to illustrate the present disclosure. In addition, the foregoing is intended to illustrate the exemplary embodiments of the present disclosure, and the present disclosure may be used in various combinations, variations, and environments. That is, changes or modifications are possible within the scope of the inventive concept disclosed in this specification, within a scope equivalent to the present disclosure and/or within the scope of skill or knowledge in the art. The embodiment of the present disclosure describes a best state for realizing the technical idea of the present disclosure, and various changes required in the specific application field and purpose of the present disclosure are also possible. Accordingly, the above detailed description of the present disclosure is not intended to limit the present disclosure to the disclosed embodiments. In addition, the appended claims should be construed to cover other embodiments as well.

## Claims

1. An augment implant to which different types of porous structures are applied, the augment implant comprising:
a body part of a porous structure which has pores which induce bone ingrowth,
wherein the body part comprises a plurality of porous parts having porosities different from each other.

2. The augment implant of claim 1, wherein the body part comprises a first porous part constituting an outer side of the body part, and a second porous part constituting an inner side of the body part.

3. The augment implant of claim 2, wherein a porosity of the first porous part is smaller than a porosity of the second porous part.

4. The augment implant of claim 3, wherein a size of a pore of the first porous part is smaller than a size of a pore of the second porous part.

5. The augment implant of claim 2, wherein a porous structure of the first porous part is formed to have an irregular shape.

6. The augment implant of claim 2, wherein a porous structure of the second porous part is formed to have a regular shape.

7. The augment implant of claim 2, wherein the augment implant further comprises a wall frame part of a nonporous structure formed along a boundary surface between the first porous part and the second porous part.

8. The augment implant of claim 2, wherein the body part comprises a through hole formed through a second surface of the body part from a first surface thereof, and
the augment implant further comprises a hole frame part of a nonporous structure formed along an inner surface of the through hole.

9. The augment implant of claim 8, wherein the hole frame part comprises a powder hole formed through a second surface of the hole frame part from a first surface thereof.

10. The augment implant of claim 9, wherein the powder hole is formed at a position at which the powder hole communicates with the second porous part of the body part.

11. The augment implant of claim 8, wherein the through hole comprises a first tapered hole which has a truncated cone-shaped space by having a diameter decreasing gradually toward a second side from a first side, and a second tapered hole which faces the first tapered hole and has a truncated cone-shaped space by having a diameter decreasing gradually toward the first side from the second side so that multidirectional fixing of a fixing means is possible.

12. The augment implant of claim 1, wherein the body part comprises an edge which is a boundary between a surface and a surface, and the augment implant further comprises an edge frame part of a nonporous structure formed along the edge.

13. The augment implant of any one of claims 1 to 12, wherein the augment implant is integrally manufactured by 3D printing.
